# DEMANDE DE BREVET EUROPEEN

(11) **EP 4 091 454 A1**
(43) Date de publication de la demande: **23.11.2022**
(21) Numéro de dépôt: 22174720.7
(22) Date de dépôt: 20.05.2022
(51) Int. Cl.: A21C 1/00, A21C 1/14, A21C 13/00, A21D 8/04, C12M 1/34, G01F 23/00, C12M 1/36

(54) **FERMENTEUR ET PROCÉDÉ POUR PRODUIRE DU LEVAIN LIQUIDE AVEC UNE VITESSE D'AGITATION VARIABLE**

(30) Priorité: 20.05.2021 BE 202105411
(71) Demandeur: JAC S.A., 4000 Liège (BE)
(72) Inventeur: VAN CAUWENBERGHE, Baudouin, 4000 Liège (BE)
(74) Mandataire: Callewaert, Koen

(57) **Abrégé**

Fermenteur et procédé pour produire du levain liquide à partir d'un mélange (16) comprenant de l'eau (14) et de la farine (15) qui sont fournis dans une cuve (2), dans lequel le mélange est agité lors de la fermentation du mélange ou entre des étapes successives de fermentation. La quantité du mélange (16) dans la cuve (2) est déterminée et l'intensité de l'agitation du mélange est adapté en fonction de la quantité du mélange (16) présent dans la cuve.

## Description

L'invention concerne un procédé pour produire du levain liquide à partir d'un mélange comprenant de l'eau et de la farine qui sont fournis dans une cuve. Ainsi, pour obtenir du levain liquide, on applique des étapes de repos successives séparées l'une de l'autre par une étape d'agitation.

Lors d'une étape d'agitation, le mélange de levain liquide est remué pour homogénéiser ce mélange. Dans une étape de repos on soumet le volume de départ du mélange à une fermentation, de sorte que le volume du levain liquide présent dans la cuve augmente.

Dans les fermenteurs à levain liquide de l'art antérieur, l'étape de repos est arrêté après l'écoulement d'un temps prédéfini et le levain est ensuite remué afin de faire descendre le niveau du levain. Après cette étape d'agitation, le mélange est à nouveau soumis à une étape de repos pour la durée prédéfinie. Cette séquence est répétée selon les prescriptions de la recette de préparation du levain liquide qui est utilisée.

Dans certains fermenteurs le mélange est agité en continu lors de la fermentation de ce mélange. Donc, il n'y a pas d'étapes séparées d'agitation et de fermentation pour la production du levain.

Des problèmes rencontrés avec les fermenteurs, suivant l'art antérieur, est que le mélange n'est pas bien homogénéisé ou que lors de l'agitation, des projections du mélange restent sur les parois de la cuve au-dessus du niveau du mélange. Ainsi ces projections sèchent et des bactéries peuvent se développer dans cette matière sèche. Finalement, les projections séchées risquent de tomber dans le mélange et de détruire le bon équilibre bactériologique du mélange.

L'invention veut remédier à cet inconvénient en proposant un procédé et un fermenteur dans lesquels un mélange homogène est obtenu sans que des projections du mélange risquent de se former sur les parois de la cuve.

Additionnellement, pour une bonne fermentation du levain et pour obtenir un levain avec une bonne qualité aromatique, il est important de minimiser l'agitation du levain. Il s'avère que trop d'agitation affecte négativement le développement des arômes et des ferments. Néanmoins, il est important d'agiter le levain de temps en temps pour homogénéiser le mélange, pour repartir la chaleur dans le liquide et pour maintenir un bon développement des ferments.

L'invention veut proposer un procédé pour optimaliser la production de levain liquide afin d'obtenir un levain d'une meilleure qualité avec une très bonne qualité aromatique. Par ailleurs, l'invention vise à proposer un procédé qui permet de préparer du levain de manière largement automatisée.

A cet effet, suivant l'invention, la quantité du mélange dans la cuve est déterminée et l'intensité de l'agitation du mélange est adapté en fonction de la quantité du mélange présent dans la cuve. Par exemple, suivant l'invention, la quantité du mélange dans la cuve est déterminée en déterminant le volume du mélange et l'intensité de l'agitation est adaptée en fonction du volume présent dans la cuve. L'intensité d'agitation est, en particulier, adaptée en fonction de ce volume de manière à minimiser les projections ou éclaboussures du mélange projetées sur les parois du réservoir au-dessus du niveau du mélange.

Avantageusement, le volume du mélange est mesuré par un capteur de niveau sans contact qui est prévu au dessus de la surface du mélange présent dans la cuve.

Suivant un forme de réalisation particulière du procédé suivant l'invention, le volume du mélange est déterminé en mesurant le niveau du mélange, en particulier le niveau de la surface supérieure de celui-ci.

De préférence, l'intensité de l'agitation du mélange, en particulier la vitesse d'agitation, est augmenté proportionnellement à l'augmentation du volume.

L'invention concerne également un fermenteur à levain comprenant une cuve pour contenir un mélange comprenant de l'eau et de la farine pour préparer du levain liquide. Ce fermenteur présente un agitateur qui permet de remuer le mélange dans la cuve et un capteur de quantité pour mesurer la quantité du mélange présent dans la cuve. L'agitateur est entrainé par des moyens d'entrainement, par exemple, un moteur électrique. Ce capteur de quantité est, par exemple, un capteur de poids pour peser le poids du mélange ou un capteur de niveau pour mesurer le niveau du mélange dans la cuve.

Le fermenteur, suivant l'invention, est caractérisé en ce qu'il comprend un dispositif de contrôle permettant de recevoir une valeur de niveau correspondant au niveau du mélange, ce dispositif coopérant avec lesdits moyens d'entrainement pour modifier l'intensité de l'agitation du mélange, en particulier la vitesse d'agitation de l'agitateur, en fonction du niveau mesuré du mélange dans la cuve.

Avantageusement, ladite valeur de niveau est mesurée par le capteur de niveau.

Préférentiellement, le dispositif de contrôle permet d'augmenter l'intensité de l'agitation du mélange proportionnellement à l'augmentation du volume de ce mélange.

Suivant une forme de réalisation intéressante du fermenteur, le capteur de niveau permet de mesurer la distance entre la surface du mélange et un niveau fixe en haut de la cuve.

D'autres détails et particularités de l'invention ressortiront de la description donnée ci-après, à titre d'exemple non limitatif, de quelques formes de réalisation particulières de l'invention avec référence aux dessins annexés.
La figure 1 est une coupe verticale schématique d'un fermenteur, suivant l'invention.
La figure 2 est une vue analogue à celle de la figure 1 avec le couvercle du fermenteur ouverte lors de la préparation d'un mélange pour préparer du levain liquide.
La figure 3 est une vue analogue à celle des figures 1 et 2 lorsque la cuve du fermenteur contient un volume de départ d'un mélange pour préparer du levain liquide.
La figure 4 est une vue analogue à celle des figures précédentes à la fin d'une étape de repos.

Dans les différentes figures, les mêmes chiffres de référence se rapportent aux mêmes éléments ou à des éléments analogues.

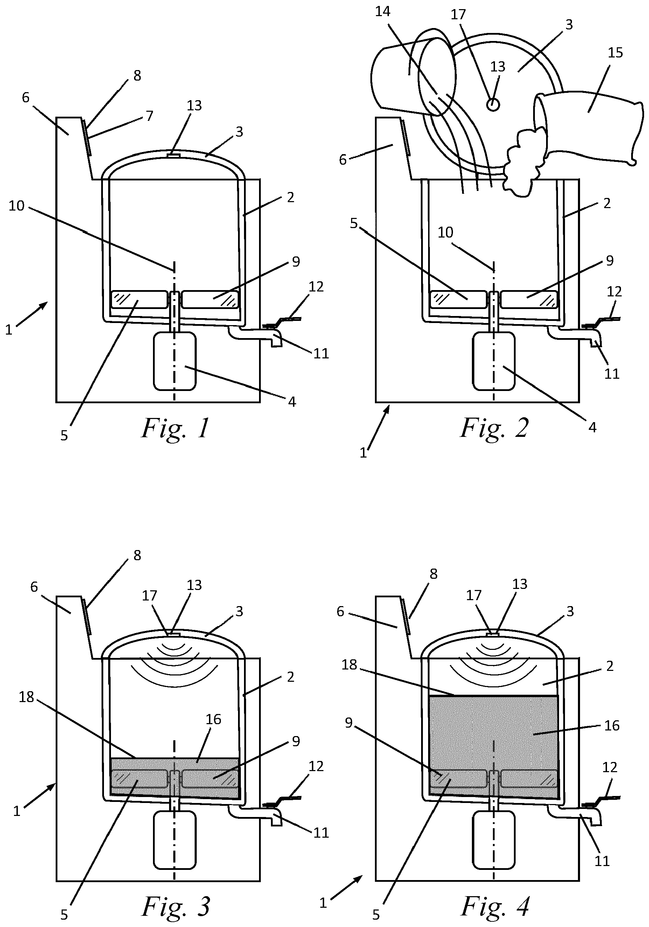

L'invention peut, en générale, être appliquée dans un procédé pour préparer du levain liquide de manière sensiblement automatisée et dans un fermenteur pour appliquer ce procédé. La figure 1 montre un tel fermenteur 1, suivant une forme de réalisation intéressante de l'invention, comprenant un châssis dans lequel sont prévus une cuve 2 avec un couvercle 3 et un moteur 4 pour entrainer un agitateur 5 présent dans la cuve 2. À sa partie supérieure le fermenteur 1 présente, généralement, un dispositif de contrôle 6 avec, par exemple, un écran 7 et des boutons de commande 8.

De préférence, la cuve 2 présente une section transversale circulaire et l'agitateur 5 comprend une ou plusieurs pales 9 qui peuvent être entrainées autour de l'axe centrale 10 de la cuve 2 pour remuer un liquide présent dans la cuve 2. Ainsi, les pales 9 sont entrainées par le moteur 4 qui est, par exemple, situé en dessous de la cuve 2.

Le fond de la cuve 2 est connecté à un tuyau 11 à travers lequel du liquide peut être prélevé de la cuve 2. Le tuyau 11 peut être fermé ou ouvert par un robinet 12 prévu à cet effet.

Suivant l'invention, le fermenteur 1 comprend un capteur de niveau pour mesurer le niveau d'un mélange présent dans la cuve 2. Dans la forme de réalisation de l'invention représentée dans les figures, ce capteur de niveau 13 est prévu dans le couvercle 3, en particulier, au milieu de celui-ci. Il est évident que ce capteur 13 peut être prévu à d'autres endroits dans le fermenteur 1. En général, le capteur de niveau 13 permet donc de déterminer le volume du mélange 16 présent dans la cuve.

Le levain liquide est préparé à partir d'un mélange comprenant de l'eau et de la farine. Comme le montre la figure 2, de l'eau 14 et de la farine 15 sont versés dans la cuve 2 du fermenteur 1 pour obtenir le mélange à partir duquel le levain est préparé. Eventuellement, des additifs supplémentaires peuvent être ajoutés à ce mélange selon les souhaits de l'opérateur pour modifier les arômes du levain.

Ensuite, l'agitateur 5 est actionné en entrainant les pales 9 autour de l'axe 10 par le moteur 4 pour mélanger et homogénéiser le contenu de la cuve 2.

Suivant une forme de réalisation particulière de l'invention, l'intensité d'agitation de l'agitateur 5, ou la vitesse de la rotation des pales 9 autour de l'axe 10, est adapté à la quantité du mélange présent dans la cuve 2. Par la quantité du mélange, on comprend le poids ou le volume du mélange. Ainsi, la quantité du mélange peut être déduite des quantités des composants du mélange, comme l'eau et la farine, pour définir l'intensité d'agitation. Lorsque la cuve 2 contient une petite quantité de mélange, la vitesse de rotation est inférieure à la vitesse de rotation qui est appliquée lorsque la cuve 2 contient une grande quantité de mélange.

Ladite petite quantité de mélange correspond, par exemple, à une quantité minimale de mélange contenu dans la cuve 2 qui est associée à une intensité d'agitation minimale et ladite grande quantité de mélange correspond, par exemple, à une quantité maximale de mélange qui est associée à une intensité d'agitation maximale. Le mélange est alors agité, lors de l'étape d'agitation, avec une intensité d'agitation comprise entre l'intensité d'agitation minimale et l'intensité d'agitation maximale qui est d'autant plus grande que le niveau du mélange est élevé par rapport au niveau minimale.

Lorsque le mélange 16 présent dans la cuve 2 est bien mélangé et homogène, l'agitateur 5 est arrêté ou sa vitesse d'entrainement est considérablement réduite.

Une étape de repos est alors appliquée dans laquelle d'abord la quantité du mélange 16, en particulier le volume du mélange 16, présent dans la cuve 2 est déterminée. Ceci est, de préférence, fait au moyen du capteur de niveau 13 sans contact qui est prévu au-dessus de la surface supérieure 18 du mélange 16 présent dans la cuve 2. Dans la forme de réalisation montrée dans les figures, ce capteur de niveau 13 comprend un capteur radar 17. Ainsi, ce capteur permet de déterminer le volume du mélange 16 présent dans la cuve 2 en mesurant la distance entre ce capteur 17 et la surface supérieure 18 du mélange 16. Donc le niveau de la surface supérieure du mélange étant mesuré, ce niveau formant ainsi un niveau de départ correspondant au volume de départ du mélange 16.

Ensuite, une valeur seuil est définie pour le volume du mélange 16, ou, en d'autres termes, pour le niveau de ce volume, correspondant au volume du mélange 16 qu'on souhait obtenir après une étape de repos.

Pour obtenir cette valeur seuil, le volume de départ est, généralement, multiplié par un facteur comprise entre 1,05 et 3. Ce facteur est normalement déterminé par la compétence de l'artisan ou par une recette pour la préparation du levain liquide. De préférence ce facteur est choisi entre 1,15 et 1,85. Dans des applications pratiques de l'invention, un facteur fixe peut éventuellement être programmé dans le dispositif de contrôle 6 du fermenteur 1.

Généralement, ladite cuve 2 présente une forme cylindrique avec une section horizontale circulaire constante. Ainsi, mesurer le niveau du mélange 16 dans la cuve 2 équivaut à la mesure du volume du mélange présent dans la cuve. Le niveau du mélange dans la cuve correspond sensiblement à la distance verticale entre le fond de la cuve 2 et la surface supérieure 18 du mélange 16 présent.

Après la détermination de la valeur seuil pour le volume du mélange 16 fermenté, le niveau de la surface supérieure 18 du mélange 16 est mesuré lors de la fermentation de celui-ci. Lors de cette fermentation, le volume du mélange 16 présent dans la cuve 2 augmente et donc le niveau du mélange 16 augmente. Le mesurage du niveau du mélange 16 peut être effectuée en continu ou en discontinu par le capteur de niveau 13. Lorsque le niveau du mélange 16 est mesuré en discontinue, le niveau est, par exemple, déterminé à des moments successifs.

Au moment où il est établi que le volume du mélange 16, ou donc le niveau de celui-ci, a atteint la valeur seuil suite à la fermentation de ce mélange, l'étape de repos est arrêtée.

Ceci est fait en commençant une étape d'agitation dans laquelle le mélange 16 fermenté est remué. En raison de cette agitation, le volume du mélange 16 diminue.

Suivant l'invention, l'intensité de l'agitation du mélange durant l'étape d'agitation est adapté en fonction de la quantité du mélange 16 présent dans la cuve 2. En particulier, en fonction du niveau mesuré, et donc en fonction du volume du mélange 16, l'intensité de l'agitation est régulée. Lorsque le niveau du mélange est relativement élevé, une intensité d'agitation est appliquée qui est supérieure à l'intensité qui serait appliquée lors d'un niveau relativement bas du mélange. Ainsi, l'intensité d'agitation est réglée afin d'éviter que le mélange ne soit pas bien homogénéisé ou que, lors de l'agitation, des projections ou des éclaboussures du mélange soient projetés sur les parois de la cuve au-dessus du niveau du mélange.

L'intensité d'agitation du mélange est déterminé par la vitesse de l'entrainement de l'agitateur 5. Donc lorsque la vitesse d'entrainement de l'agitateur 5, en particulier de la pale 9, autour de l'axe central 10, augmente, l'intensité d'agitation augmente également.

L'étape d'agitation est suivie d'une nouvelle étape de repos comprenant déterminer le volume du mélange 16 dans la cuve 2, ce volume formant un nouveau volume de départ, et soumettre le mélange 16 à une fermentation pour que le volume du mélange 16 augmente à nouveau jusqu'à une valeur seuil qui est déterminée en fonction du nouveau volume de départ du mélange. Cette valeur seuil est obtenue par multiplier le nouveau volume de départ avec le facteur susdit.

Ainsi, dans le procédé pour produire du levain liquide à partir du mélange de l'eau 14 et de la farine 15, on applique des étapes de repos successives séparées l'une de l'autre par une étape d'agitation jusqu'à l'obtention d'un levain liquide qui peut être utilisé dans la préparation de produits de viennoiserie et de boulangerie, comme le pain.

Lorsque la production de levain liquide est terminée, la cuve 2 peut éventuellement être refroidie pour bloquer la fermentation du mélange pour stocker le levain.

Le levain liquide peut être soutirée de la cuve 2 à travers le tuyau 11 en ouvrant le robinet 12 jusqu'à ce que la quantité désirée soit obtenue.

Il est important de maintenir une quantité minimale de levain liquide dans la cuve 2. Cette quantité minimale peut servir de mélange de base pour la production de levain dans un procédé de production ultérieur. Ainsi, le niveau du mélange 16 présent dans la cuve 2 est mesuré lorsque du levain liquide est soutirée de la cuve. Ceci est, par exemple, fait par le capteur de niveau 13. Lorsqu'une valeur minimale prédéterminée du niveau du mélange 16 est atteinte dans la cuve 2, le robinet 12 est fermé automatiquement ou une alarme est déclenchée pour indiquer que le soutirage du levain liquide doit être terminé.

Comme expliqué ci-dessus, une intensité d'agitation minimale peut être associée à cette quantité minimale de mélange contenu dans la cuve.

La quantité minimale de levain qui est conservé dans la cuve 2 est choisie, par exemple, en fonction du volume de levain qu'on veut produire ultérieurement dans le fermenteur 1. De la farine et de l'eau sont ajoutés au levain restant dans la cuve pour obtenir un nouveau mélange pour la production de levain liquide dans le fermenteur 1 suivant le procédé de l'invention décrit ci-dessus.

Donc le niveau minimale de la quantité de levain liquide à conserver dans la cuve 2 est ajustable par l'opérateur et peut, par exemple, être introduit manuellement dans le dispositif de contrôle 6 afin de déclencher une alarme lorsque ce niveau prédéterminé est atteint par le volume du mélange et détecté par le capteur de niveau 13.

Le fermenteur 1 peut également comprendre un capteur de contact prévu dans la paroi de la cuve 2 à un niveau qui correspond à un volume minimal de levain qui doit être conservé pour assurer le bon fonctionnement du fermenteur 1 lors de production subséquente de levain.

De manière analogue, un capteur de contact, en particulier un capteur de débordement, est éventuellement prévu dans la partie supérieure de la paroi de la cuve 2. Lorsque le niveau du mélange atteint ce capteur, une alarme est déclenchée ou une étape d'agitation est entamée pour empêcher que le liquide déborde la cuve 2. Il va de soi que, alternativement, le capteur de niveau sans contact 13 peut également être utilisé comme capteur de débordement.

La présence du capteur de niveau 13 permet de mesurer la vitesse de montée de la surface du mélange 16 lors de l'étape de repos. Lorsque cette vitesse dépasse une valeur seuil prédéterminée, ladite étape de repos est terminée par le démarrage d'une étape d'agitation.

Le capteur de niveau 13 détermine le volume du mélange 16 en mesurant la distance entre la surface supérieure 18 du mélange 16 et une référence, en particulier un niveau fixe au-dessus du mélange 16. Ce niveau fixe correspond, par exemple, à la position du capteur 13. Ce capteur de niveau 13 peut être formé par n'importe quel capteur qui permet de mesurer le niveau du mélange ou, en général, de déterminer le volume du mélange, comme par exemple un capteur radar 17, un capteur optique, un capteur ultrason, etc. De préférence le capteur 13 est un capteur non-contact qui est prévu à une distance du mélange et au-dessus de celui-ci. Ce capteur permet de mesurer le niveau du mélange par rapport à ledit niveau fixe ou par rapport au niveau de départ du mélange. Ainsi, il est possible de mesurer le niveau du volume du mélange par rapport à ce niveau fixe ou un changement du niveau du mélange par rapport au niveau de départ de celui-ci.

Le fermenteur à levain, suivant l'invention, se caractérise par la présence du dispositif de contrôle 6 qui permet de recevoir une valeur correspondant au niveau de départ du mélange 13.

Ainsi, cette valeur peut être introduit dans le dispositif de contrôle 6 par l'opérateur après que celui-ci a préparé le mélange de départ. A cet effet, le dispositif de contrôle présent un moyen d'entrée, comme par exemple un écran tactile 7 ou des boutons de commande 8, pour entrer ladite valeur de départ dans le dispositif de contrôle 6. Il est également possible que l'opérateur introduit directement la valeur seuil pour le volume du mélange, ou le niveau de celui-ci, qu'on souhaite obtenir à la fin de l'étape de repos.

Mais, de préférence cette valeur du niveau ou du volume de départ du mélange 16, provenant du capteur de niveau 13, est saisie automatiquement par le dispositif de contrôle 6 suite au coopération entre le capteur 13 et le dispositif de contrôle 6. A partir de cette valeur provenant du capteur 13, une valeur seuil est alors générée par le dispositif de contrôle 6 qui correspond au volume fermenté souhaité, ou au niveau de ce volume souhaité, comme décrit plus haut.

Le dispositif de contrôle 6 coopère alors avec le capteur 13 pour mesurer le niveau du mélange lors de l'étape de repos et pour comparer ladite valeur seuil avec la mesure du niveau par le capteur 13. Lorsque qu'il est établi que cette valeur seuil est atteint, le dispositif de contrôle 6 déclenche l'entrainement de l'agitateur 5 afin de terminer l'étape de repos et de démarrer l'étape d'agitation. L'intensité de l'agitation est réglée en fonction du niveau mesuré du mélange.

Le dispositif de contrôle 6 est également capable de stocker une valeur minimale pour le niveau du mélange 16 dans la cuve 2. Lorsque cette valeur minimale est atteinte par le mélange 16 comme déterminé au moyens du capteur de niveau 13, le dispositif de contrôle génère une alarme ou déclenche la fermeture automatique d'une vanne d'évacuation 12 par laquelle la cuve 2 peut être vidée.

Néanmoins que, dans la description donnée ci-dessus, l'intensité d'agitation est réglée en fonction du volume du mélange présent dans la cuve 2, il est également possible de régler l'intensité d'agitation en fonction du poids du mélange. Ainsi, le poids du mélange peut être introduit dans le dispositif de contrôle par l'opérateur ou le poids du contenu de la cuve est mesuré automatiquement par un capteur qui coopère avec le dispositif de contrôle 6.

Il est bien entendu que l'invention n'est pas limitée aux différentes formes de réalisation décrites ci-dessus, mais que d'autres variantes encore peuvent être envisagées sans sortir du cadre de la présente invention, notamment en ce qui concerne la forme de la cuve 2 et la présence et la position de capteurs. Il est, par exemple, possible que autour de la cuve, des moyens de refroidissement ou de réchauffement sont prévus qui coopèrent avec des capteurs thermiques. Le dispositif de contrôle 6 peut, par exemple, comprendre des moyens pour introduire ou pour activer de cycles préprogrammés d'une succession d'étapes d'agitation et de fermentation.

## Revendications

1. Procédé pour produire du levain liquide à partir d'un mélange (16) comprenant de l'eau (14) et de la farine (15) qui sont fournis dans une cuve (2), dans lequel le mélange est agité lors de la fermentation du mélange ou entre des étapes successives de fermentation, **caractérisé en ce que** la quantité du mélange (16) dans la cuve (2) est déterminée et l'intensité de l'agitation du mélange est adapté en fonction de la quantité du mélange (16) présent dans la cuve de manière à ce que l'intensité de l'agitation appliquée à une petite quantité de mélange est inférieure à l'intensité de l'agitation appliquée à une grande quantité de mélange.

2. Procédé suivant la revendication 1, dans lequel la quantité du mélange est déterminée en mesurant le volume du mélange (16) dans la cuve (2).

3. Procédé suivant la revendication 2, dans lequel la quantité du mélange (16) est mesurée par un capteur de niveau (13) sans contact qui est prévu au dessus de la surface (18) du mélange (16) présent dans la cuve (2).

4. Procédé suivant la revendication 3, dans lequel le capteur de niveau (13) sans contact comprend un capteur radar (17).

5. Procédé suivant l'une quelconque des revendications 2 à 4, dans lequel la quantité du mélange (16) est déterminée en mesurant le niveau du mélange (16), en particulier le niveau de la surface supérieure (18) de celui-ci.

6. Procédé suivant l'une quelconque des revendications 2 à 5, dans lequel la quantité du mélange (16) est mesurée en déterminant la distance, ou le changement de distance, entre la surface du mélange (16) et un niveau fixe au-dessus du mélange (16) ou entre la surface du mélange (16) et un niveau de départ du mélange (16).

7. Procédé suivant l'une quelconque des revendications 2 à 6, dans lequel l'intensité de l'agitation du mélange, en particulier la vitesse d'agitation, est augmenté proportionnellement à l'augmentation du volume lors de l'étape d'agitation.

8. Procédé suivant la revendication 1, dans lequel la quantité du mélange est déterminée en mesurant le poids du mélange (16) présent dans la cuve (2).

9. Procédé suivant l'une quelconque des revendications 1 à 8, dans lequel une intensité d'agitation minimale est associée à une quantité minimale de mélange contenu dans la cuve (2) et une intensité d'agitation maximale est associée à une quantité maximale de mélange, le mélange étant agité lors de l'étape d'agitation avec une intensité d'agitation comprise entre l'intensité d'agitation minimale et l'intensité d'agitation maximale qui est d'autant plus grande que le niveau du mélange est élevé par rapport au niveau minimale.

10. Fermenteur à levain comprenant une cuve (2) pour contenir un mélange comprenant de l'eau et de la farine pour préparer du levain liquide, le fermenteur (1) comprenant un agitateur (5) permettant de remuer le mélange présent dans la cuve (2) et un capteur de quantité pour mesurer la quantité du mélange dans la cuve (2), ledit agitateur (5) étant entrainé par des moyens d'entrainement, **caractérisé en ce que** le fermenteur (1) comprend un dispositif de contrôle permettant de recevoir une valeur de niveau correspondant à la quantité du mélange (16) présent dans la cuve (2), ce dispositif coopérant avec lesdits moyens d'entrainement pour modifier l'intensité de l'agitation du mélange (16), en particulier la vitesse d'agitation de l'agitateur (5), en fonction de ladite valeur de niveau de manière à ce que l'intensité de l'agitation appliquée à une petite quantité de mélange est inférieure à l'intensité de l'agitation appliquée à une grande quantité de mélange.

11. Fermenteur suivant la revendication 10, dans laquelle ledit capteur de quantité est un capteur de niveau (13) permettant de mesurer le niveau du mélange dans la cuve (2), ladite valeur de niveau correspondant au niveau du mélange (16) mesuré par le capteur de niveau (13),

12. Fermenteur suivant la revendication 10 ou 11, dans lequel le dispositif de contrôle permet d'augmenter l'intensité de l'agitation du mélange proportionnellement à l'augmentation du volume de ce mélange.

13. Fermenteur suivant l'une quelconque des revendications 10 à 12, dans lequel le capteur de niveau (13) comprend un capteur de niveau sans contact, comme par exemple un capteur radar (17), un capteur ultrason ou un capteur optique.

14. Fermenteur suivant l'une quelconque des revendications 10 à 13, dans lequel le capteur de niveau (13) permet de mesurer la distance entre la surface (18) du mélange (16) et un niveau fixe en haut de la cuve (2).

15. Fermenteur suivant l'une quelconque des revendications 10 à 14, dans lequel le capteur de quantité comprend un capteur de poids permettant de mesurer le poids du mélange (16) comprenant un moyen d'entrée (7,8) pour entrer le poids mesuré du mélange (16) dans le dispositif de contrôle (6).

16. Fermenteur suivant l'une quelconque des revendications 10 à 15, dans lequel le dispositif de contrôle permet de stocker une intensité d'agitation minimale associée à une quantité minimale du mélange contenu dans la cuve et une intensité d'agitation maximale associée à une quantité maximale du mélange contenu dans la cuve, ce dispositif coopérant avec lesdits moyens d'entrainement pour appliquer une intensité d'agitation, comprise entre l'intensité d'agitation minimale et l'intensité d'agitation maximale, qui est d'autant plus grande que la quantité du mélange est élevée par rapport à la quantité minimale en fonction de ladite valeur de niveau.
